# FASCICULE DE BREVET EUROPEEN

(11) **EP 2 675 463 B1**
(45) Date de publication et mention de la délivrance du brevet: **05.08.2015**
(21) Numéro de dépôt: 12704432.9
(22) Date de dépôt: 20.02.2012
(51) Int. Cl.: A61K 33/00, A61K 35/08, A61K 36/61, A61K 36/534, A61P 27/14

(54) **SOLUTION IONIQUE AQUEUSE CONTENANT DE L'EAU DE MER ET AU MOINS UN COMPOSE ORIGINELLEMENT NON MISCIBLE A L'EAU DE MER**
WÄSSRIGE IONISCHE LÖSUNG MIT MEERWASSER UND MINDESTENS EINER, URSPRÜNGLICH MIT MEERWASSER UNMISCHBAREN VERBINDUNG
AQUEOUS IONIC SOLUTION CONTAINING SEAWATER AND AT LEAST ONE COMPOUND THAT IS ORIGINALLY IMMISCIBLE WITH SEAWATER

(30) Priorité: 18.02.2011 FR 1151357
(43) Date de publication de la demande: 25.12.2013
(73) Titulaire: Laboratoire de la Mer, 35400 Saint-Malo (FR)
(72) Inventeur: ANDRO, François, F-35630 Saint-Symphorien (FR); BEAULIEU, Anne, F-35800 Dinard (FR)
(74) Mandataire: Maillet, Alain
(86) Numéro de dépôt international: PCT/EP2012/052886
(87) Numéro de publication internationale: WO 2012/110665

(56) Documents cités:
- WO-A1-2004/024122
- WO-A1-2008/037938
- WO-A2-2005/053638
- FR-A1- 2 777 187
- FR-A1- 2 915 389
- US-A- 5 948 414
- US-A1- 2006 045 849
- US-A1- 2006 210 482
- DATABASE GNPD [Online] Mintel; février 2011 (2011-02), anonymous: "Eucalyptus nasal & sinus congestion relief", XP002658089, extrait de www.gnpd.com Database accession no. 1486027
- DATABASE GNPD [Online] Mintel; décembre 2010 (2010-12), anonymous: "Decongestant nasal spray", XP002658090, extrait de www.gnpd.com Database accession no. 1453962
- DATABASE GNPD [Online] Mintel; mars 2011 (2011-03), anonymous: "Seawater nasal hygiene spray", XP002658091, extrait de www.gnpd.com Database accession no. 1510042
- DATABASE GNPD [Online] Mintel; avril 2010 (2010-04), Anonymous: "Purifying nasal spray", XP002658092, extrait de www.gnpd.com Database accession no. 1313166
- DATABASE GNPD [Online] Mintel; janvier 2009 (2009-01), Anonymous: "Decongestive nasal spray", XP002658093, extrait de www.gnpd.com Database accession no. 1024003
- DATABASE GNPD [Online] Mintel; avril 2005 (2005-04), Anonymous: "Cleansing & gentle foam", XP002658094, extrait de www.gnpd.com Database accession no. 355563
- FOST D ET AL: "A novel strategy for achieving efficacy in deodorants", COSMETICS & TOILETRIES, WHEATON, IL, US, vol. 122, no. 10, 1 octobre 2007 (2007-10-01), pages 63-70, XP009091841, ISSN: 0361-4387

## Description

### 1. Domaine de l'invention

Le domaine de l'invention est celui des solutions à base d'eau de mer, utilisées notamment dans le traitement de muqueuses, notamment nasales ou plus généralement de la sphère otorhinolaryngée, ou de la peau.

### 2. Art antérieur

On connaît dans l'art antérieur de nombreuses solutions à base d'eau de mer.

Par «solution à base d'eau de mer », on entend dans la présente description toute solution contenant plus de 25% en poids d'eau de mer, de préférence plus de 75% en poids d'eau de mer, qui présente une osmolarité comprise entre 250 mOsm/kg et 350 mOsm/kg, notamment entre 290 mOsm/kg et 315 mOsm/kg. L'osmolarité recherchée peut être obtenue soit par déplétion de l'eau de mer en certaines espèce ioniques, soit en diluant l'eau de mer avec de l'eau pure.

Cette définition n'englobe pas les solutions dites sérums physiologiques ou salines. En effet, ces solutions sont dénommées ainsi car elles sont isotoniques. Toutefois, elles contiennent à titre d'espèces ioniques uniquement des ions Na et Cl. Les solutions salines ou sérums physiologiques sont donc très différents de l'eau de mer, de par leur composition ionique et la quantité d'ions contenue.

La société demanderesse a déjà décrit l'application de solutions ioniques isoosmotiques à base d'eau de mer pour prévenir et limiter la libération des médiateurs chimiques responsables du déclenchement des phénomènes inflammatoires des muqueuses bronchiques et pulmonaires, notamment dans le brevet EP 1 091 747, mais également pour un traitement céruménolytique, dans le brevet EP 1 091 746. La demanderesse a également décrit l'usage de l'eau de mer pour le traitement de la congestion nasale grâce à des solutions hypertoniques dans le brevet EP 2 068 896.

Par ailleurs, la société demanderesse a développé des solutions ioniques aqueuses du genre en question, enrichies en potassium pour une utilisation pour le traitement et le lavage de l'oeil ainsi que pour une utilisation en tant que produit de rinçage de lentilles de contact. Ces solutions et leurs utilisations sont décrites notamment dans les brevets FR 2 843 029 et FR 2 803 205.

L'eau de mer présente l'avantage de contenir de nombreux oligo-éléments d'origine naturelle, indispensables au maintien de l'équilibre cellulaire. Les solutions développées à base d'eau de mer, ci-après dénommées « solutions à base d'eau de mer » peuvent être isotoniques (avec une osmolarité comprise entre 250 mOsm/kg et 350 mOsm/kg environ) ou hypertoniques (dont l'osmolarité est supérieure à 350 mOsm/kg). Les solutions isotoniques sont notamment utilisées pour leurs propriétés hydratantes, réparatrices et anti-inflammatoires. Les solutions hypertoniques sont notamment utilisées pour décongestionner les muqueuses pro-inflammatoires et fluidifier les sécrétions produites.

Pour renforcer ou diversifier les propriétés d'une solution à base d'eau de mer, il peut être intéressant d'ajouter à la solution des composés ayant d'autres propriétés préventives ou curatives.

Le brevet FR 2 569 536 propose pour la préparation de solutions nutritives parentérales de lier eau de mer et vitamines par de la lécithine. Cet émulsifiant est largement utilisé en industrie alimentaire pour obtenir des émulsions liant eau et lipides.

La demande de brevet EP 1 074 245 propose d'associer des extraits végétaux aux sels de la Mer Morte pour des applications dermatologiques.

### 3. Inconvénients de l'art antérieur

Certains composés d'origine naturelle ou chimique qu'il serait intéressant d'associer à des solutions à base d'eau de mer, compte tenu de leurs propriétés bienfaisantes à l'égard de la peau ou des muqueuses, sont toutefois non miscibles dans de telles solutions.

Cette non miscibilité peut résulter notamment de la force osmotique naturelle de l'eau de mer et/ou du caractère lipophile de tels composés.

Ainsi, de nombreux extraits de plantes, notamment les huiles essentielles, mais aussi certaines vitamines sont originellement non miscibles aux solutions d'eau de mer. Certains extraits aqueux de plantes sont également non miscibles à de telles solutions à base d'eau de mer de par la force osmotique naturelle de l'eau de mer.

En pratique, l'association d'une solution à base d'eau de mer et de tels composés originellement non miscibles à une telle solution conduit à des compositions instables qui ne sont pas des solutions mais au mieux des émulsions qui forment plus ou moins rapidement des précipités, et/ou des émulsions instables qui déphasent.

De plus, les compositions obtenues grâce à de telles associations peuvent présenter des pH atteignant des valeurs non physiologiques, c'est-à-dire des pH soit inférieurs à 7 soit supérieurs à 8,4, qui les rendent non utilisables ou difficilement utilisables pour une utilisation notamment dans la sphère otorhinolaryngée.

### 4. Objectifs de l'invention

L'invention a notamment pour objectif de permettre l'association, en une solution ionique aqueuse, d'une solution à base d'eau de mer et d'au moins un composé originellement non miscible à une telle solution à base d'eau de mer.

Notamment, un objectif de l'invention est de proposer une telle solution ionique aqueuse qui soit stable, c'est-à-dire qui ne déphase pas et qui ne forme pas de précipités.

Encore un autre objectif est de proposer une telle solution ionique qui présente, dans au moins certains modes de réalisation, un pH compris entre 7 et 8,4.

Encore un autre objectif est de divulguer une telle solution ionique qui, dans au moins certains modes de réalisation, soit applicable par pulvérisation ou nébulisation.

Un autre objectif est de fournir une telle composition qui, dans au moins certains modes de réalisation, soit limpide.

Un autre objectif est d'utiliser une telle composition qui, dans au moins certains modes de réalisation, puisse être utilisée pour traiter à titre préventif ou curatif la sphère otorhinolaryngée, notamment les muqueuses nasales et/ou pour traiter à titre préventif ou curatif la zone dermatologique, et/ou pour traiter à titre préventif ou curatif la zone gastro-intestinale, et/ou pour traiter à titre préventif ou curatif les troubles liés à la nutrition.

L'objectif de proposer, dans au moins un de ses modes de réalisation, une solution ionique aqueuse à usage nasal, comprenant une solution à base d'eau de mer et au moins un composé originellement non miscible à une telle solution à base d'eau de mer, est également décrit.

L'objectif de proposer un procédé de préparation d'une telle composition est également décrit.

### 5. Exposé de l'invention

Tout ou partie de ces objectifs sont atteints grâce à, conformément à l'invention, une solution ionique aqueuse, destinée notamment à un usage nasal, comprenant au moins:
- une solution à base d'eau de mer présentant une osmolalité comprise, pour une solution isotonique entre 250 mOsm/kg et 350 mOsm/kg, et pour une solution hypertonique au dessus de 350 mOsm/kg, et plus particulièrement entre 350 mOsm/kg et 1074 mOsm/kg, dans une proportion massique comprise entre 25% et 98% environ par rapport à la masse totale de la composition,
- au moins un phospholipide cationique d'origine naturelle dans une proportion massique comprise entre 0,01% et 5% environ par rapport à la masse totale de ladite solution ionique aqueuse,
- au moins un composé originellement non miscible à ladite solution à base d'eau de mer dans une proportion massique comprise entre 0,01% et 10% environ par rapport à la masse totale de ladite solution ionique aqueuse, ledit composé originellement non miscible à ladite solution à base d'eau de mer étant choisi parmi les extraits végétaux huileux.

Selon l'invention, la présence dans une solution ionique aqueuse d'un phospholipide cationique d'origine naturelle permet la solubilisation du composé originellement non miscible dans une solution à base d'eau de mer.

Les inventeurs ont en effet découvert de façon surprenante que les phospholipides cationiques d'origine naturelle avaient un pouvoir solubilisant permettant d'associer une solution à base d'eau de mer et un composé originellement non miscible à cette solution à base d'eau de mer pour former une solution ionique aqueuse conforme à l'invention. De plus, le recours à un solubilisant d'origine naturelle permet de limiter l'irritation et la sensibilisation des muqueuses otorhynolaryngées par des produits chimiques. Ce point est particulièrement important lorsque ces muqueuses sont fragilisées par une inflammation, une infection, une réaction allergique ou la pollution environnementale.

De telles solutions selon l'invention sont stables. Elles peuvent également être très limpides. On entend par une solution stable une solution qui lorsque dans un laps de temps déterminé, ses propriétés essentielles ne changent pas ou changent au plus dans des proportions tolérables. De plus il est entendu que la solution doit être conservée dans des conditions appropriées et prescrites de température, d'humidité et d'exposition à la lumière et qu'un récipient convenable a été utilisé.

La stabilité des propriétés suivantes est recherchée :
- Stabilité chimique : teneur en principes actifs compris entre 95 et 105%,
- Stabilité physique : aspect, goût
- Stabilité microbiologique : contamination, prolifération
- Stabilité thérapeutique : effet thérapeutique inchangé
- Stabilité toxicologique : pas d'augmentation de la toxicité (produits de dégradation) (Pharmacopée Helvétique, 9^{ème} édition).

La limpidité d'une solution peut se mesurer par mirage : examinée dans les conditions appropriées elle doit être exempte de particules visibles à l'oeil nu. (PE 2.9.20). La limpidité peut aussi se mesurer par densité optique à l'aide d'un spectrophotomètre (PE 2.2.25 et PE 2.2.23).

Les solutions ioniques aqueuses selon l'invention présentent aussi l'avantage d'être applicables par pulvérisation ou nébulisation.

Une solution ionique aqueuse conforme à l'invention associant solution à base d'eau de mer et au moins un composé originellement non miscible peut permettre de renforcer les propriétés bienfaisantes de la solution à base d'eau de mer.

De telles propriétés peuvent être notamment être des propriétés d'hydratation de la peau ou des muqueuses, des propriétés réparatrices de la peau ou des muqueuses, des propriétés anti-inflammatoires de la peau et des muqueuses, des propriétés fluidifiantes des mucosités nasales et/ou encore des propriétés décongestionnantes des muqueuses nasales.

Une solution ionique aqueuse conforme à l'invention peut permettre de combiner, voire de potentialiser, les propriétés de la solution à base d'eau de mer et celles du composé originellement non miscible, notamment dans le traitement des affections de la sphère otorhinolaryngée et du tractus respiratoire.

Un autre avantage de la composition conforme à l'invention est de permettre d'augmenter la durée d'action de la solution à base d'eau de mer, grâce à la présence du composé originellement non miscible.

Un autre avantage de la composition conforme à l'invention peut être de permettre de limiter le recours aux spécialités médicamenteuses, tels que les vasoconstricteurs ou les antiseptiques.

Un autre avantage de la composition conforme à l'invention est de permettre d'améliorer l'observance des patients du fait de la sensation agréable pour le patient.

En particulier, la solution ionique aqueuse selon l'invention peut comprendre :
- ladite solution à base d'eau de mer présentant une osmolalité comprise entre 290 mOsm/kg et 350 mOsm/kg, notamment comprise entre 290 mOsm/kg et 315 mOsm/kg dans une proportion massique comprise entre 90% et 98% environ par rapport à la masse totale de ladite solution ionique aqueuse,
- ledit au moins un phospholipide cationique d'origine naturelle, dans une proportion massique comprise entre 0,10% et 0,20% environ par rapport à la masse totale de ladite solution ionique aqueuse,
- ledit au moins un composé originellement non miscible à ladite solution à base d'eau de mer dans une proportion massique comprise entre 0,10% et 0,30% environ par rapport à la masse totale de ladite solution ionique aqueuse.

La solution à base d'eau de mer peut être telle que celles décrites dans les documents de brevet FR-A-2915389, EP-A-1091747, EP-A-1091746, FR-A-2843029, EP-A- 2 068 896 et FR-A-2803205.

Ledit au moins un composé originellement non miscible à la solution à base d'eau de mer peut être choisi dans le groupe constitué par : les extraits huileux de plantes.

Ledit composé originellement non miscible dans la solution à base d'eau de mer peut être choisi en fonction de la destination de la composition, par exemple en fonction de l'effet thérapeutique ou préventif désiré. Il peut être choisi par exemple parmi des extraits végétaux, notamment des huiles essentielles végétales comme l'huile essentielle de ravinsara, l'huile essentielle de menthe, l'huile essentielle de sauge, l'huile essentielle de citron, l'huile essentielle de thym, l'huile essentielle de romarin, l'huile essentielle de pin, l'huile essentielle de gaulthérie, l'huile essentielle de myrte, l'huile essentielle de myrrhe... En particulier, ledit composé originellement non miscible à ladite solution à base d'eau de mer peut comprendre un extrait huileux de menthe, d'eucalyptus et/ou de niaouli.

Un composé originellement non miscible dans la solution à base d'eau de mer choisi parmi les vitamines, notamment A, D, E et K, ou les acides gras comme l'huile d'amande douce, l'huile d'olive, l'huile de noyau d'abricot est également décrit. On peut également citer à titre d'exemple de composé originellement non miscible à la solution à base d'eau de mer les hydratants tels que la cire d'abeille et la glycérine.

Le phospholipide cationique d'origine naturelle contient préférentiellement le myristamidopropyl propylène glycol dimonium chloride phosphate, lequel peut être avantageusement extrait de la noix de coco. Le phospholipide cationique d'origine naturelle peut également comprendre la phosphatidylcholine et/ou les phospholipides cationiques issus du lait.

La solution ionique aqueuse selon l'invention peut en outre comprendre un conservateur naturel dans une proportion massique comprise entre 0,01% et 10% environ par rapport la masse totale de ladite solution ionique aqueuse, ledit conservateur naturel étant apte à résister à une stérilisation en milieu humide à 120°C pendant 20 min environ. Ledit conservateur naturel peut notamment être dans une proportion massique comprise entre 0,10% et 0,80% environ par rapport la masse totale de ladite solution ionique aqueuse. Ledit conservateur naturel peut comprendre par exemple un homopolymère de L-Lysine.

La solution ionique aqueuse selon l'invention ne comprend de préférence pas de conservateur chimique, dont l'utilisation répétée ou prolongée peut être nocive à la physiologie notamment de la muqueuse nasale. En effet, le conservateur chimique le plus utilisé actuellement est le chlorure de benzalkonium. Or, il a été décrit dans la littérature que ce conservateur d'origine chimique induit des effets délétères sur la muqueuse nasale. Plus précisément, le chlorure de benzalkonium est considéré comme toxique pour les neutrophiles humains, même à faible concentration (Boston et al, 2003, Arch. Otolaryngolo Head Neck Surgery, vol. 129, 660-664). Cet effet du chlorure de benzalkonium sur les muqueuses nasales a été observé chez des patients même pour une courte utilisation (GrafP.et al, 1999, Arch. Otolaryngolo Head Neck Surgery, vol. 125, 1128-1132). La composition selon l'invention permet donc de résoudre ce problème en incorporant un conservateur naturel.

La solution ionique aqueuse peut comprendre en outre un composé rafraîchissant, dans une proportion massique comprise entre 1% et 10% environ, notamment entre 1% et 4% environ, par rapport à la masse totale de ladite solution ionique aqueuse. Selon un mode de réalisation, ledit composé rafraîchissant peut comprendre du sorbitol, du xylitol, du menthol...

La solution ionique aqueuse conforme à l'invention peut par exemple être utilisée pour la fabrication de lotions, de lingettes, de pulvérisateurs ou autres.

L'invention a encore pour objet, en combinaison avec ce qui précède, l'utilisation de la solution ionique aqueuse telle que définie plus haut pour le traitement des muqueuses nasales.

L'invention a encore pour objet, en combinaison avec ce qui précède, l'utilisation de la solution ionique aqueuse telle que définie plus haut pour la fabrication d'un médicament ou d'un produit à usage pharmaceutique destiné à prévenir ou à traiter les affections de la sphère otorhinolaryngée (ORL) choisies parmi, le rhume commun, la rhinosinusite aiguë, la sinusite aiguë, la rhinosinusite chronique, la rhinite allergique, l'otite, la rhinopharyngite, la pharyngite, la laryngite, la trachéite, la bronchite,

L'utilisation de la solution ionique aqueuse telle que définie plus haut pour la fabrication d'un médicament ou d'un produit à usage pharmaceutique destiné à prévenir ou à traiter les affections dermatologiques, les affections de la sphère gastro-intestinale et/ou les affections relatives à la nutrition est également décrite. Le procédé de préparation de la solution ionique aqueuse telle que définie plus haut est également décrit.

Le procédé de préparation de la solution ionique aqueuse selon l'invention peut être le suivant :
on prépare, comme décrit ci-après, une solution à base d'eau de mer présentant une osmolalité comprise entre 250 mOsm/kg et 350 mOsm/kg, notamment entre 290 mOsm/kg et 315 mOsm/kg environ, l'osmolalité pouvant être choisie en fonction de l'utilisation recherchée de la solution ionique aqueuse.

On homogénéise chacune des matières premières constituées par le ou les solubilisants, le ou les composés originellement non miscibles à l'eau de mer et le ou les composés rafraîchissants, le cas échéant.

On pèse ces matières premières.

On met dans une cuve un premier solubilisant, puis, incorporés sous agitation, un deuxième solubilisant le cas échéant le ou les composés originellement non miscibles, tels que des huiles essentielles, éventuellement le conservateur naturel et éventuellement le composé rafraîchissant ou arôme. On mélange pendant 15 min environ.

On assemble ce mélange de matières premières à la solution à base d'eau de mer et on mélange pendant environ 30 min pour former la solution ionique aqueuse conforme à l'invention.

Avantageusement, le premier solubilisant est le Myristamidopropyl propyleneglycol dimonium chloride phosphate et le second solubilisant, du sorbitol. L'utilisation du sorbitol comme second solubilisant permet d'éviter d'ajouter des concentrations en Myristamidopropyl propyleneglycol trop massives, ce qui pourrait se révéler irritant pour les muqueuses fragiles.

### 6. Exemples

### 6.1. Solution hypertonique à base d'eau de mer aux huiles essentielles de menthe et d'eucalyptus.

On a réalisé une solution ionique aqueuse présentant la composition suivante (proportions massiques par rapport à la masse totale de la solution ionique aqueuse) :
- Solution à base d'eau de mer, hypertonique aqueuse, à teneur en sels de 22g/l, électrodyalisée: 97,54%
- Epsilon-polylysine (polypeptide dont les lysines sont liées entre elles par des liaisons epsilon) : 0,2%
- Sorbitol : 0,16%
- Myristamidopropyl propyleneglycol dimonium chloride phosphate : 2%
- Arôme menthe-eucalyptus LN 05614/1 (42,2% vol.) : 0,1%
Dans cet exemple, l'arôme contient en volume :
- de l'alcool éthylique à 96%,
- du monopropylèneglycol,
- de l'huile essentielle d'eucalyptus : 0,042%, et
- du menthol cristallisé extrait de *Mentha arvensis* : 0,0112%

Pour réaliser cette solution ionique aqueuse, on a procédé de la manière suivante.

On a préparé, par électrodialyse, une solution à base d'eau de mer à teneur en sel de 22g/l, présentant une osmolalité comprise entre 310 mOsm/kg et 325 mOsm/kg pour la solution isotonique, et au delà de 350 mOsm/kg pour une solution hypertonique. Le procédé de réalisation de cette solution à base d'eau de mer, connue de l'art antérieur, est rappelé ci-dessous.

On a homogénéisé chacune des matières premières constituées dans cet exemple par de l'epsilon-polylysine, de Myristamidopropyl propyleneglycol dimonium chloride phosphate, du sorbitol et de l'arôme menthe-eucalyptus.

On a pesé chacune de ces matières premières, et on a disposé le Myristamidopropyl propyleneglycol dimonium chloride phosphate dans une cuve, puis, sous agitation, l'epsilon-polylysine, le sorbitol et l'arôme menthe-eucalyptus.

Après avoir mélangé l'ensemble pendant environ 15 min, on l'a ajouté à la solution à base d'eau de mer pour former la solution ionique aqueuse souhaitée après mélange pendant environ 30 min. La solution est parfaitement limpide.

### 6.2. Solution hypertonique à base d'eau de mer aux huiles essentielles de menthe, d'eucalyptus et de niaouli.

On prépare une solution ionique aqueuse présentant la composition suivante (proportions massiques par rapport à la masse totale de la solution ionique aqueuse) :
- Solution à base d'eau de mer, hypertonique aqueuse, à teneur en sels de 22g/l, électrodyalisée: 90%
- Epsilon-polylysine (polypeptide dont les lysines sont liées entre elles par des liaisons epsilon) : 8%
- Sorbitol : 1,55%
- Myristamidopropyl propyleneglycol dimonium chloride phosphate : 0,1%
- Arôme menthe-eucalyptus LN 05614/1 (42,2% vol.) : 0,2%
- Huile essentielle de niaouli (*Melaleuca quinquerrervia*) : 0,1%
- Alcool éthylique 96% PE (Ethanol 96 surfin) : 0,05%
Dans cet exemple, l'arôme contient en volume :
- de l'alcool éthylique à 96%,
- du monopropylèneglycol,
- de l'huile essentielle d'eucalyptus : 0,042%, et
- du menthol cristallisé extrait de *Mentha arvensis* : 0,0112%

Cette solution a été préparée selon le même mode opératoire décrit dans l'exemple précédent. La solution est parfaitement limpide.

### 6.3. Solution hypertonique à base d'eau de mer aux huiles essentielles de thym et de citron.

On prépare une solution ionique aqueuse présentant la composition suivante (proportions massiques par rapport à la masse totale de la solution ionique aqueuse) :
- Solution à base d'eau de mer, hypertonique aqueuse, à teneur en sels de 22g/l, électrodyalisée: qsp 100%
- Sorbitol (Neosorb 70/70B) : 2%
- Myristamidopropyl propyleneglycol dimonium chloride phosphate : 0,3%
- Huile essentielle *Thymus zygis* L : 0,1%
- Huile essentielle de citron : 0,054%

Cette solution a été préparée selon le même mode opératoire décrit dans l'exemple précédent. La solution est parfaitement limpide.

### 7. La solution à base d'eau de mer

Tel que décrit dans la demande de brevet FR-A-2915389, la solution à base d'eau de mer utilisée dans les formulations ci-dessus, qui est une solution ionique isoosmotique à base d'eau de mer peut présenter :
- un pH compris entre 7,6 et 8,4,
- une teneur en matière sèche de 1 à 2% en poids,
- une osmolalité comprise entre 250 et 400 mOsm/kg, notamment entre 250 mOsm/kg et 350 mOsm/kg environ,
- de 500 à 2600 mg/l de sodium (Na),
- de 40 à 6500 mg/l de potassium (K),
- de 5800 à 7000 mg/l de chlorure (Cl),
- de 20 à 400 mg/l de calcium (Ca),
- de 50 à 1500 mg/l de magnésium (Mg).

La solution à base d'eau de mer peut être stérile ou être stérilisée.

La solution à base d'eau de mer peut comprendre d'autres éléments, tels que du fer (Fe), du zinc (Zn), du cuivre (Cu), du manganèse (Mn), du sélénium (Se).

La solution à base d'eau de mer est préparée par électrodialyse de l'eau de mer.

Plus particulièrement, successivement :
- on prélève, en tant que matière première, de l'eau de mer, d'une teneur en sels supérieure ou égale à 32g/l, de préférence par une profondeur de 5 à 10 mètres dans une zone à forts mouvements de courant,
- on analyse et clarifie cette eau,
- on désode l'eau décantée par électrodialyse jusqu'à l'obtention d'une osmolalité comprise entre 290 mOsm/kg et 350 mOsm/kg, notamment entre 290 mOsm/kg et 315 mOsm/kg pour une solution isotonique et au delà de 350 mOsm/kg, et plus particulièrement entre 350 mOsm/kg et 1074 mOsm/kg, pour une solution hypertonique,
- on ajuste les concentrations ioniques par électrodialyse sélective,
- on filtre et on stocke le produit éventuellement dans des conditions stériles.

Dans toute la présente description, l'expression « comprenant un » doit être comprise comme étant synonyme de l'expression « comprenant au moins un », sauf si le contraire est spécifié.

Dans toute la présente description, les plages de valeurs doivent être comprises comme incluant les bornes, sauf si le contraire est spécifié.

### 8. Contre-Exemples

Des essais effectués dans les mêmes conditions mais en utilisant d'autres types de solubilisants en lieu et place d'un phospholipide cationique d'origine naturelle selon l'invention ont conduit à des compositions instables ne formant pas des solutions ou présentant des inconvénients les rendant inutilisables dans le cadre des applications envisagées. La limpidité a été mesurée par densité optique à l'aide d'un spectrophotomètre (PE 2.2.25 et PE 2.2.23).

Par exemple, le solubilisant DISPER®, qui est un complexe émulsionnant pour les huiles essentielles, flocule au contact de l'eau de mer. Le surfactant SoluBol® tend à précipiter et ne remplit par conséquent plus son rôle. Le MontanOV® donne une solution trouble tandis que l'utilisation de glycérine comme solubilisant donne une solution opalescente. Or il est nécessaire d'obtenir une solution parfaitement limpide pour le plaisir d'utilisation. En revanche, les compositions préparées conformément aux exemples indiqués dans la présente demande sont parfaitement limpides.

## Revendications

1. Solution ionique aqueuse, notamment destinée à un usage nasal, comprenant au moins:
- une solution à base d'eau de mer présentant une osmolalité comprise entre 250 mOsm/kg et 350 mOsm/kg pour une solution isotonique ou une osmolalité supérieure à 350 mOsm/kg pour une solution hypertonique dans une proportion massique comprise entre 25% et 98% environ par rapport à la masse totale de ladite solution ionique aqueuse,
- au moins un phospholipide cationique d'origine naturelle dans une proportion massique comprise entre 0,01% et 5% environ par rapport à la masse totale de ladite solution ionique aqueuse,
- au moins un composé originellement non miscible à ladite solution à base d'eau de mer dans une proportion massique comprise entre 0,01% et 10% environ par rapport à la masse totale de ladite solution ionique aqueuse, ledit composé originellement non miscible à ladite solution à base d'eau de mer étant choisi parmi les extraits végétaux huileux.

2. Solution ionique aqueuse selon la revendication 1, **caractérisée en ce qu'**elle comprend au moins:
- ladite solution à base d'eau de mer présentant une osmolalité comprise entre 290 mOsm/kg et 350 mOsm/kg pour une solution isotonique ou une osmolalité supérieure à 350 mOsm/kg pour une solution hypertonique, dans une proportion massique comprise entre 90% et 98% environ par rapport à la masse totale de ladite solution ionique aqueuse,
- ledit au moins un phospholipide cationique d'origine naturelle, dans une proportion massique comprise entre 0,10% et 0,20% environ par rapport à la masse totale de ladite solution ionique aqueuse,
- ledit au moins un composé originellement non miscible à ladite solution à base d'eau de mer dans une proportion massique comprise entre 0, 10% et 0,30% environ par rapport à la masse totale de ladite solution ionique aqueuse.

3. Solution ionique aqueuse selon l'une des revendications 1 et 2, **caractérisée en ce que** ledit au moins un composé originellement non miscible à ladite solution à base d'eau de mer est une huile essentielle.

4. Solution ionique aqueuse selon l'une quelconque des revendications 1 à 3, **caractérisée en ce qu'**elle comprend un conservateur naturel dans une proportion massique comprise entre 0,01% et 10% environ par rapport la masse totale de la composition, ledit conservateur naturel étant apte à résister à une stérilisation en milieu humide à 120°C pendant 20 min environ.

5. Solution ionique aqueuse selon la revendication 4, **caractérisée en ce que** ledit conservateur naturel est dans une proportion massique comprise entre 0,10% et 0,80% environ par rapport la masse totale de la composition.

6. Solution ionique aqueuse selon l'une des revendications 4 et 5, **caractérisée en ce que** ledit conservateur naturel comprend un homopolymère de L-Lysine.

7. Solution ionique aqueuse selon l'une quelconque des revendications 1 à 6, **caractérisée en ce qu'**elle comprend un composé rafraîchissant, dans une proportion massique comprise entre 1% et 10% environ, notamment entre 1% et 4% environ, par rapport à la masse totale de la composition.

8. Solution ionique aqueuse selon la revendication 7, **caractérisée en ce que** ledit composé rafraîchissant comprend du sorbitol.

9. Composition ionique aqueuse selon l'une quelconque des revendications 1 à 8, **caractérisée en ce que** ledit composé originellement non miscible à ladite solution à base d'eau de mer comprend un extrait huileux de menthe et d'eucalyptus.

10. Composition ionique aqueuse selon l'une quelconque des revendications 1 à 9, **caractérisée en ce que** ledit phospholipide cationique d'origine naturelle est le myristamidopropyl propylène glycol dimonium chloride phosphate, la phosphatidylcholine et/ou les phospholipides cationiques issus du lait.

11. Solution ionique aqueuse telle que définie dans l'une quelconque des revendications 1 à 10 pour son utilisation pour prévenir ou traiter les affections de la sphère otorhinolaryngée choisies parmi le rhume commun, la rhinosinusite aiguë, la sinusite aiguë, la rhinosinusite chronique, la rhinite allergique, l'otite, la rhinopharyngite, la pharyngite, la laryngite, la trachéite ou la bronchite.

## Patentansprüche

1. Wässrige ionische Lösung, die insbesondere zur nasalen Verwendung bestimmt ist, mindestens umfassend:
- eine Lösung auf der Basis von Meerwasser, die eine Osmolalität im Bereich zwischen 250 mOsm/kg und 350 mOsm/kg für eine isotonische Lösung oder eine Osmolalität von mehr als 350 mOsm/kg für eine hypertonische Lösung in einem Massenanteil im Bereich zwischen etwa 25 % und etwa 98 %, bezogen auf die Gesamtmasse der wässrigen ionischen Lösung, aufweist,
- mindestens ein kationisches Phospholipid natürlichen Ursprungs in einem Massenanteil im Bereich zwischen etwa 0,01 % und etwa 5 %, bezogen auf die Gesamtmasse der wässrigen ionischen Lösung,
- mindestens eine ursprünglich mit der Lösung auf der Basis von Meerwasser unmischbare Verbindung in einem Massenanteil im Bereich zwischen etwa 0,01 % und etwa 10 %, bezogen auf die Gesamtmasse der wässrigen ionischen Lösung, wobei die ursprünglich mit der Lösung auf der Basis von Meerwasser unmischbare Verbindung ausgewählt ist aus Ölauszügen aus Pflanzen.

2. Wässrige ionische Lösung nach Anspruch 1, **dadurch gekennzeichnet, dass** die mindestens umfasst:
- die Lösung auf der Basis von Meerwasser, die eine Osmolalität im Bereich zwischen 290 mOsm/kg und 350 mOsm/kg für eine isotonische Lösung oder eine Osmolalität von mehr als 350 mOsm/kg für eine hypertonische Lösung in einem Massenanteil im Bereich zwischen etwa 90 % und etwa 98 %, bezogen auf die Gesamtmasse der wässrigen ionischen Lösung, aufweist,
- das mindestens eine kationische Phospholipid natürlichen Ursprungs in einem Massenanteil im Bereich zwischen etwa 10 % und etwa 0,20 %, bezogen auf die Gesamtmasse der wässrigen ionischen Lösung,
- die mindestens eine ursprünglich mit der Lösung auf der Basis von Meerwasser unmischbare Verbindung in einem Massenanteil im Bereich zwischen etwa 0,10 % und etwa 0,30 %, bezogen auf die Gesamtmasse der wässrigen ionischen Lösung.

3. Wässrige ionische Lösung nach einem der Ansprüche 1 und 2, **dadurch gekennzeichnet, dass** die mindestens eine ursprünglich mit der Lösung auf der Basis von Meerwasser unmischbare Verbindung ein etherisches Öl ist.

4. Wässrige ionische Lösung nach einem der Ansprüche 1 bis 3, **dadurch gekennzeichnet, dass** sie ein natürliches Konservierungsmittel in einem Massenanteil im Bereich zwischen etwa 0,01 % und etwa 10 %, bezogen auf die Gesamtmasse der Zusammensetzungen, umfasst, wobei das natürliche Konservierungsmittel geeignet ist, gegen eine etwa 20-minütige Sterilisation in wässrigem Medium bei 120 °C beständig zu sein.

5. Wässrige ionische Lösung nach Anspruch 4, **dadurch gekennzeichnet, dass** das natürliche Konservierungsmittel in einem Massenanteil im Bereich zwischen etwa 0,10 % und etwa 0,80 %, bezogen auf die Gesamtmasse der Zusammensetzung, vorliegt.

6. Wässrige ionische Lösung nach einem der Ansprüche 4 und 5, **dadurch gekennzeichnet, dass** das natürliche Konservierungsmittel ein L-Lysin-Homopolymer umfasst.

7. Wässrige ionische Lösung nach einem der Ansprüche 1 bis 6, **dadurch gekennzeichnet, dass** sie eine erfrischende Verbindung in einem Massenanteil im Bereich zwischen etwa 1 % und etwa 10 %, insbesondere zwischen etwa 1 % und etwa 4 %, bezogen auf die Gesamtmasse der Zusammensetzung, umfasst.

8. Wässrige ionische Lösung nach Anspruch 7, **dadurch gekennzeichnet, dass** die erfrischende Verbindung Sorbitol umfasst.

9. Wässrige ionische Zusammensetzungen nach einem der Ansprüche 1 und 8, **dadurch gekennzeichnet, dass** die ursprünglich mit der Lösung auf der Basis von Meerwasser unmischbare Verbindung einen Ölauszug von Minze und Eukalyptus umfasst.

10. Wässrige ionische Zusammensetzung nach einem der Ansprüche 1 bis 9, **dadurch gekennzeichnet, dass** das kationische Phospholipid natürlichen Ursprungs Myristamidopropyl-Propylenglycol-Dimoniumchloridphosphat, Phosphatidylcholin und/oder die aus Milch stammenden kationischen Phospholipide sind.

11. Wässrige ionische Lösung, wie in einem der Ansprüche 1 bis 10 definiert, für ihre Verwendung zur Verhütung oder Behandlung von Erkrankungen des Hals-Nasen-Ohren-Bereichs, ausgewählt aus gewöhnlichem Schnupfen, akuter Rhinosinusitis, aktuter Sinusitis, chronischer Rhinosinusitis, allergischer Rhinitis, Otitis, Rhinopharyngitis, Pharyngitis, Laryngitis, Tracheitis oder Bronchitis.

## Claims

1. Aqueous ionic solution, especially intended for nasal use, comprising at least:
- a solution based on seawater with an osmolality of between 250 mOsm/kg and 350 mOsm/kg for an isotonic solution or an osmolality of greater than 350 mOsm/kg for a hypertonic solution, in a proportion by weight of between approximately 25% and 98% relative to the total weight of said aqueous ionic solution,
- at least one cationic phospholipid of natural origin in a proportion by weight of between approximately 0.01% and 5% relative to the total weight of said aqueous ionic solution,
- at least one compound that is originally immiscible with said solution based on seawater, in a proportion by weight of between approximately 0.01% and 10% relative to the total weight of said aqueous ionic solution, said compound that is originally immiscible with said solution based on seawater being selected from plant oil extracts.

2. Aqueous ionic solution according to Claim 1, **characterized in that** it comprises at least:
- said solution based on seawater with an osmolality of between 290 mOsm/kg and 350 mOsm/kg for an isotonic solution or an osmolality of greater than 350 mOsm/kg for a hypertonic solution, in a proportion by weight of between approximately 90% and 98% relative to the total weight of said aqueous ionic solution,
- said at least one cationic phospholipid of natural origin in a proportion by weight of between approximately 0.10% and 0.20% relative to the total weight of said aqueous ionic solution,
- said at least one compound that is originally immiscible with said solution based on seawater, in a proportion by weight of between approximately 0.10% and 0.30% relative to the total weight of said aqueous ionic solution.

3. Aqueous ionic solution according to either of Claims 1 and 2, **characterized in that** said at least one compound that is originally immiscible with said solution based on seawater is an essential oil.

4. Aqueous ionic solution according to any one of Claims 1 to 3, **characterized in that** it comprises a natural preservative in a proportion by weight of between approximately 0.01% and 10% relative to the total weight of the composition, said natural preservative being able to resist sterilization in a moist environment at 120°C for approximately 20 min.

5. Aqueous ionic solution according to Claim 4, **characterized in that** said natural preservative is in a proportion by weight of between approximately 0.10% and 0.80% relative to the total weight of the composition.

6. Aqueous ionic solution according to either of Claims 4 and 5, **characterized in that** said natural preservative comprises a homopolymer of L-lysine.

7. Aqueous ionic solution according to any one of Claims 1 to 6, **characterized in that** it comprises a cooling compound in a proportion by weight of between approximately 1% and 10%, especially between approximately 1% and 4%, relative to the total weight of the composition.

8. Aqueous ionic solution according to Claim 7, **characterized in that** said cooling compound comprises sorbitol.

9. Aqueous ionic composition according to any one of Claims 1 to 8, **characterized in that** said compound that is originally immiscible with said solution based on seawater comprises a mint and eucalyptus oil extract.

10. Aqueous ionic composition according to any one of Claims 1 to 9, **characterized in that** said cationic phospholipid of natural origin is myristamidopropyl propylene glycol-dimonium chloride phosphate, phosphatidylcholine and/or milk-derived cationic phospholipids.

11. Aqueous ionic solution as defined in any one of Claims 1 to 10, for use thereof for preventing or treating ear, nose and throat disorders selected from the common cold, acute rhinosinusitis, acute sinusitis, chronic rhinosinusitis, allergic rhinitis, otitis, rhinopharyngitis, pharyngitis, laryngitis, tracheitis or bronchitis.
